(19) Europäisches Patentamt
European Patent Office
Office européen des brevets

(11) Publication number: **0 024 122**
**B1**

(12) **EUROPEAN PATENT SPECIFICATION**

(45) Date of publication of patent specification: **16.02.83**

(51) Int. Cl.³: **C 07 D 473/34, A 61 K 31/52**

(21) Application number: **80302475.1**

(22) Date of filing: **22.07.80**

(54) **Adenine derivatives possessing pharmacological activity.**

(30) Priority: **25.07.79 GB 7925959**

(43) Date of publication of application: **25.02.81 Bulletin 81/8**

(45) Publication of the grant of the patent: **16.02.83 Bulletin 83/7**

(84) Designated Contracting States: **BE CH DE FR GB IT LI NL SE**

(56) References cited:
**DE - A - 2 549 410**
**DE - A - 2 716 837**
**US - A - 3 836 656**

**Chemical Abstracts vol. 89, no. 19, 6 November 1978 Columbus, Ohio, USA. W. S. DIMENNA et al. "Synthesis of potential hypolipidemic agents. Reaction of substituted phenyl 2,3-epoxypropyl ethers with adenine, uracil and thymine" page 17, column 1, abstract no. 157160n.**

(73) Proprietor: **BEECHAM - WUELFING GmbH & Co. KG**
**Stresemannallee 6 P.O. Box 25**
**D-4040 Neuss (DE)**

(72) Inventor: **Jozic, Ljerka**
**An Der Bismarck-Schule 4 B**
**D-3000 Hannover 1 (DE)**
Inventor: **Tauscher, Manfred**
**An Der Beeke 21**
**D-3212 Gronau (Leine) (DE)**

(74) Representative: **Russell, Brian John et al,**
**European Patent Attorney Beecham Pharmaceuticals Great Burgh Yew Tree Bottom Road**
**Epsom Surrey, KT18 5XQ (GB)**

(56) References cited:
**Alfred Burger, Medicinal Chemistry third edition, part I, 1970 Wiley Interscience New York, page 75.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 024 122 B1**

Courier Press, Leamington Spa, England

Adenine derivatives possessing pharmacological activity

The Journal of Medicinal Chemistry, *21*, (10), 1073—76 (1978) discloses 9-(p-chlorophenoxy-2-hydroxypropyl)adenine as a hypolipidaemic.

GB—A—1530166 (equivalent to DE—A1—2716837) discloses that certain adenine derivatives are able to potentiate antihyperlipidaemics, inter alia 5-methylpyrazole-2-carboxylic acid. It has now been found that certain other adenine derivatives are active in their own right as antihyperlipidaemic agents.

The present invention provides the compounds of the formulae (I) and (II):

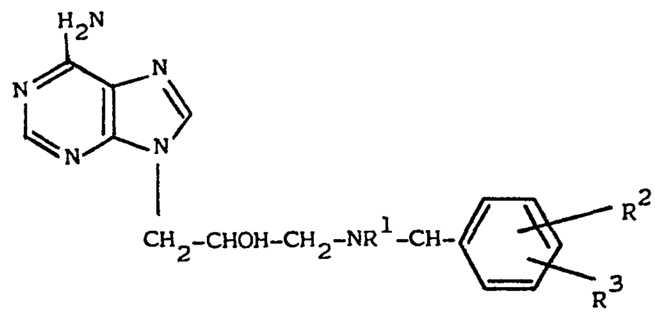

(I)

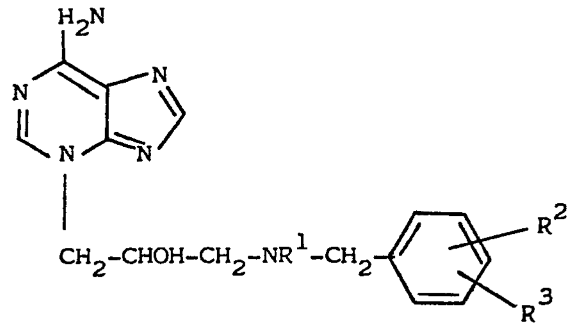

(II)

and salts thereof wherein $R^1$ is a hydrogen atom or a $C_{1-4}$ alkyl group; $R^2$ is a moiety selected from hydrogen, fluorine, chlorine and bromine atoms and $C_{2-4}$ alkyl, $C_{1-4}$ alkoxyl, $C_{1-4}$ alkanoyloxy, $C_{1-4}$ alkoxycarbonyl and trifluoromethyl groups; and $R^3$ is a moiety selected from hydrogen, fluorine and chlorine atoms and $C_{1-4}$ alkyl and $C_{1-4}$ alkoxyl groups.

Suitable values for $R^1$ include the hydrogen atom and the methyl, ethyl, n-propyl, iso-propyl and n-butyl groups, in particular iso-propyl.

Apt values for $R^2$ include the hydrogen, fluorine, chlorine and bromine atoms and the methyl, methoxyl, acetoxyl, methoxycarbonyl, ethoxycarbonyl and trifluoromethyl groups.

Apt values for $R^3$ include hydrogen, fluoro, chloro, methyl and methoxyl.

A favoured value for $R^3$ is the hydrogen atom.

Favoured values for $R^2$ include the hydrogen and chlorine atoms.

A particularly apt value for the $C_6H_3R^2R^3$ moiety is the phenyl group. A further particularly apt value for the $C_6H_3R^2R^3$ moiety is the 2-chlorophenyl group.

Of the compounds of the formulae (I) and (II) compounds of the formula (I) are preferred.

It will of course be realised that the compounds of the formula (I) and (II) have a chiral centre at the —CHOH— group, and are thus capable of existing in enantiomeric forms.

The invention extends to each of the enantiomers of the compounds of the formulae (I) and (II) and to racemates thereof.

The compounds of this invention may be provided in the form of solvates such as hydrates.

The salts of the compounds of the formulae (I) and (II) include acid addition salts and these are preferably acid addition salts with pharmaceutically acceptable acids. Such acids may be inorganic or organic acids such as hydrochloric, hydrobromic, orthophosphoric, sulphuric, methanesulphonic, acetic, citric, lactic, tartaric, propionic, benzoic and fumaric acids.

The salts of the compounds of the formulae (I) and (II) also include pharmaceutically acceptable quaternary ammonium salts. Examples of such salts include such compounds quaternised by compounds such as $R_5$—Y wherein $R_5$ is $C_{1-6}$ alkyl, phenyl —$C_{1-6}$ alkyl or $C_{5-7}$ cycloalkyl, and Y is an anion of an acid. Suitable examples of $R_5$ include methyl, ethyl and *n*- and *iso*-propyl; and benzyl and phenylethyl. Suitable examples of Y include the halides such as chloride, bromide and iodide.

Crystalline acid addition salts are particularly apt.

Examples of compounds of the formula (I) include 6-amino-9-[2-hydroxy-3-(N-benzyl-N-isopropylamino)propyl]purine and 6-amino-9-[2-hydroxy-3-(N-2′-chlorobenzylamino)propyl]purine.

The present invention also provides a pharmaceutical composition which comprises a compound of this invention or a pharmaceutically acceptable salt thereof as hereinbefore described together with a pharmaceutically acceptable carrier.

The compositions of this invention may be adapted for administration by injection if required but it is normally preferred that they are in a form suitable for oral administration. Oral dosage forms may contain conventional excipients such as fillers, lubricants, disintegrants, binders, preservatives, colourants and so on. Suitable fillers include lactose, microcrystalline cellulose, calcium phosphate, mannitol and the like. Suitable lubricants include magnesium stearate and stearic acid. Suitable disintegrants include polyvinylpolypyrrolidone and sodium starch glycollate.

The oral dosage forms are normally provided as discrete unit forms such as tablets and capsules. In general such unit dosage forms will contain from 5 to 500 mgs and more usually from 25 to 300 mgs of the compound of formula (I) or (II). These unit dosage forms may be administered from 1 to 6 times daily in such a way that the daily dose for a 70 kg adult will normally be between 30 to 1500 mgs and more usually from 100 to 1000 mgs, for example from 200 to 800 mgs.

In another aspect this invention provides a process for the preparation of a compound of this invention which process comprises

a. the reaction of adenine or a metal salt thereof with a compound of the formula (III):

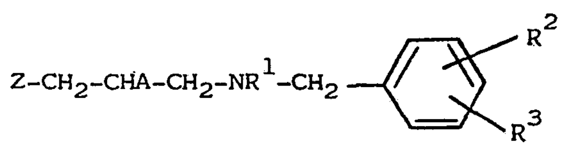

(III)

wherein:

$R^1$, $R^2$ and $R^3$ are as defined in formulae (I) and (II); and

A is hydroxyl and Z is a group readily displaceable by a nucleophile; or

A and Z together form an oxide diradical;

b. the reaction of a compound of the formula (IV) or (V):

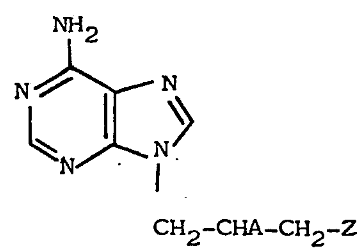

(IV)

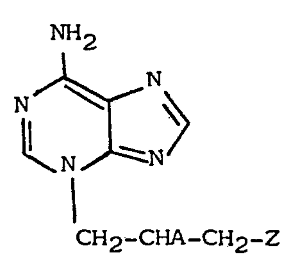

(V)

wherein:

A and Z are as hereinbefore defined with a compound of the formula (VI):

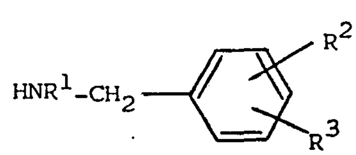

(VI)

wherein:

$R^1$, $R^2$ and $R^3$ are as defined in formulae (I) and (II);

c. the reaction of a compound of the formula (VII) or (VIII):

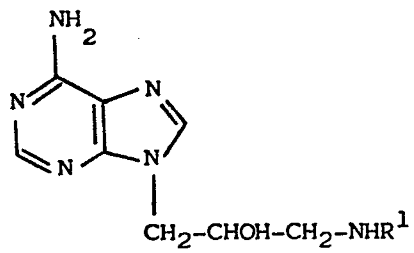

(VII)

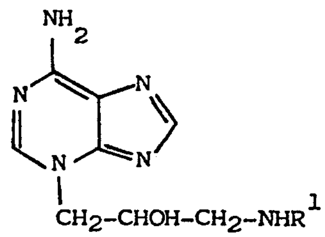

(VIII)

wherein $R^1$ is as hereinbefore defined, with a compound of the formula (IX):

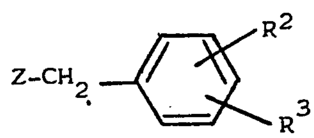

(IX)

wherein Z, $R^2$ and $R^3$ are as defined in formula (III);

d. the reduction of a compound of the formula (X) or (XI):

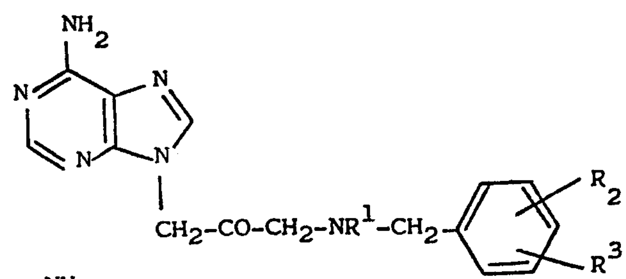

(X)

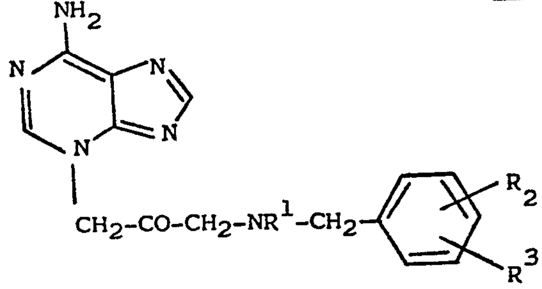

(XI)

wherein $R^1$, $R^2$ and $R^3{}_2$ are as defined in formulae (I) and (II);

e. for a compound of the formula (I), the reaction of formamide with a compound of the formula (XII):

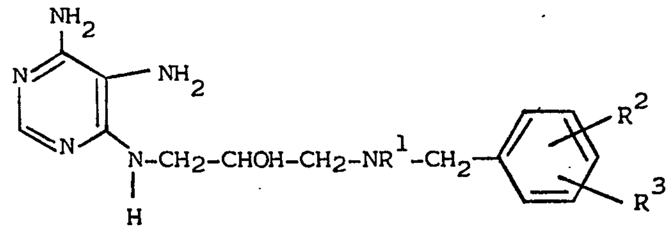

(XII)

wherein $R^1$, $R^2$ and $R^3$ are as defined in formulae (I) and (II); and optionally thereafter salifying the compound of the formula (I) or (II) so produced; or converting $R^1$ when hydrogen to a $C_{1-4}$ alkyl group.

Process variant a is a preferred process of the invention.

In process variants a, b and c suitable examples of Z when a group readily displaceable by a nucleophile include halogen atoms such as bromine or chlorine, and activated ester residues such as methanesulphonyloxy or toluenesulphonyloxy.

If adenine per se is employed in process variant a then an inorganic base for example an alkali metal base such as the hydroxide or carbonate, for example potassium hydroxide or carbonate, is used to form a metal salt in situ; alternatively, a pre-formed metal salt, for example the potassium salt, is used.

The reaction of process variant a results in the preparation of a mixture of compounds of the formula (I) and (II) (c.f. J.H. Lister, "The Chemistry of Heterocyclic Compounds; Fused Pyrimidines, Part II (Purines)" (Wiley-Interscience, 1971), and this process thus also comprises the optional separation of the compounds of formulae (I) and (II) by conventional means. If both salification and separation are carried out, then they may be carried out in any appropriate order.

Separation of the compounds of the formulae (I) and (II) may be effected by fractional crystallisation or by column chromatography, for example over silica using a solvent such as chloroform or ethyl acetate optionally in the presence of a lower alkanol such as methanol.

It will be apparent that optional salification appropriately follows separation when by chromatography.

Reaction conditions of process variants b and c are substantially those for variant a. However, the presence of base is optional, but preferred when Z is halogen. In variant b excess compound of formula (VI) may be used as an alternative base.

For process variant d, suitable examples of reducing agents are those which do not affect the optionally substituted benzyl group in the compounds of the formula (VI) or (VII). Such agents include borohydrides, such as sodium or lithium borohydrides, which may be used in a solvent such as diglyme, a lower alcohol or an ether, at non-extreme temperatures, such as ambient temperature.

Process variant e is suitably carried out analogously to the methods of Bredereck et al., given in Chem. Ber., *86*, 333 (1953) and U.K. Patent No. 706,424.

The different enantiomeric forms of compounds of the formula (I) or (II) may be resolved by the usual methods, or any given isomer may be obtained by asymmetric synthesis, for example using a given enantiomeric form of a compound of the formula (III), (VII), (VIII) or (XII) or (IV) or (V) when A is hydroxyl.

Racemates of these compounds may be resolved conventionally, e.g. by salification with a chiral acid and separation of the resultant salts, for example as in L.F. Fieser and M. Fieser, 'Organic Chemistry', 3rd Ed., 1956, Reinhold; S.H. Wilen 'Tables of Resolving Agents and Optical Resolutions', University of Notre Dame Press, 1972, or S.H. Wilen, 'Topics in Stereochemistry', 1971, Vol, 6, John Wiley, NY.

The free bases of the compounds of the formulae (I) and (II) may be salified by treatment with an acid, or treatment with a compound $R_5Y$ as hereinbefore defined.

In general, within acid addition salts, di-salts are preferred, since they are more easily prepared using a slight excess of the relevant acid.

It will also be realised that salts of the compounds of the formulae (I) and (II) which are not pharmaceutically acceptable may be useful as intermediates in the preparation of pharmaceutically acceptable salts of compounds of the formulae (I) and (II) or the compounds of the formulae (I) and (II) themselves, and so such form an aspect of the present invention.

The biological activity of the compounds of this invention are illustrated by the following test:

The hypocholesterolaemic and hypotriglyceridaemic effects of 6-amino-9-[2-hydroxy-3-N-benzyl-N-isopropylamino)propyl]purine (Compound A) and 6-amino-9-[2-hydroxy-3-(N-o-chlorobenzyl-amino)propyl]purine (Compound B) were assessed as follows. Groups of 8 male rats (Sprague-Dawley from Tuck's) weighing 80—100 g were given a powdered commercially available diet (Oxid) to which compounds were added at the level of 0.1%. These diets were fed for 7 days. The rats were then killed and the serum total cholesterol and triglyceride as well as their high-density lipoprotein (HDL)-cholesterol were measured by a Technicon AutoAnalyser. The results are expressed as percentage change from control values.

| Compound | Serum Cholesterol | Serum Triglyceride | Liver wt. as % body wt. |
|---|---|---|---|
| A | −25 | −52 | +3 |
| B | −32 | −61 | +2 |

No drug induced deaths occur at the therapeutic dose.

The following Examples illustrate the invention, and the following Descriptions illustrate the preparation of intermediates therefor:

Example 1

6-Amino-9-[2-hydroxy-3-(N-benzyl-N-isopropyl-amino)-propyl]-purine

In a 1000 ml three necked round bottomed flask, 34.6 g (0.2 mol) adenine-potassium salt are suspended in 20 ml isopropanol and the solution is refluxed. Then 0.2 moles of 1-chloro-2-hydroxy-3-(N-isopropyl-N-benzyl)aminopropane are dropped in under stirring. After 4 hours the reaction is finished. The hot reaction mixture is filtered by suction. The precipitate $P_0$ consists of potassium chloride and polar side products. The solution is cooled and precipitate $P_1$ is suctioned. $P_1$ shows similar ingredients as $P_0$. The resulting solution is treated with 25 ml ethyl acetate to precipitate the remaining side products as $P_2$. The filtrate is evaporated to dryness and the residue recrystallised from acetonitrile. To remove polar side products, the crystals are dissolved in acetonitrile and boiled with 15 g silica gel. The hot solution is filtered and cooled and the crystalline precipitate is filtered by suction and dried to yield the title compound (21.4 g), m.p. 144°C.

| Analysis: | C | H | N | O |
|---|---|---|---|---|
| calcd: | 63.51 | 7.11 | 24.68 | 4.70 |
| found: | 63.48 | 7.04 | 24.92 | 4.63 |

Example 2

6-Amino-9-[2-hydroxy-3-(N-2′-chlorobenzyl-amino)-propyl]-purine

In a 250 ml three necked round bottomed flask, 1.38 g (0.008 mol) adenine-potassium salt are refluxed with 35 ml methanol. 1-Chloro-2-hydroxy-3-(N-o-chlorobenzyl)aminopropane is diluted with 20 ml methanol and dropped in slowly under stirring. The reaction is finished after 8 hours. The methanol is removed under reduced pressure and the residue is boiled in isopropanol and the hot solution filtered. The isopropanol solution is evaporated to dryness and the residue resolved in chloroform. This chloroform solution is filtered over 30 g silica gel, which is eluted with a chloroform/methanol mixture. The obtained pure product is recrystallised from ethylacetate to yield 300 mg, m.pt. 131°C.

| Analysis: | C | H | N | O | Cl |
|---|---|---|---|---|---|
| calcd: | 54.14 | 5.15 | 25.25 | 4.80 | 10.65 |
| found: | 54.12 | 5.10 | 25.26 | 5.32 | 10.13 |

*Description 1*

1-Chloro-2-hydroxy-3-(N-isopropyl-N-benzyl)-amino-propane

37.3 g N-isopropyl-benzylamine are diluted with 200 ml isopropanol in a 500 ml round bottomed three necked flask and 23.1 g epichlorohydrin are dropped in very slowly under stirring. The temperature is kept at 20—25°C. The reaction mixture is stirred over a period of 24 hours, then 1 ml epichlorohydrin is added and stirred for a further 3 hours.

*Description 2*

1-Chloro-2-hydroxy-3-(N-o-chlorobenzyl)-amino-propane

1.41 g o-chlorobenzylamine are dissolved in 5 ml isopropanol in a 100 ml round bottomed three necked flask and 0.93 g epichlorohydrin are dropped in very slowly under stirring. The reaction mixture is heated to 30—40°C to shorten the reaction time. After 24 hours the reaction is finished. A white precipitate (~500 mg) is recombined with its mother liquor after TLC control.

**Claims**

1. A compound of the formula (I) or (II):

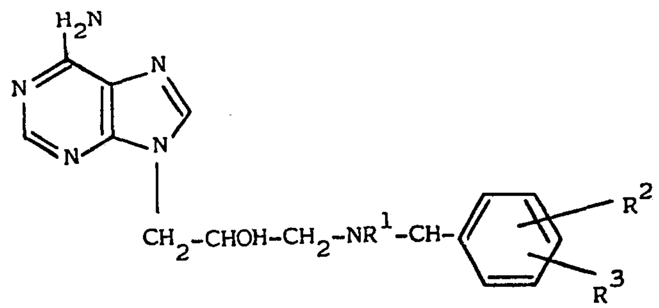

(I)

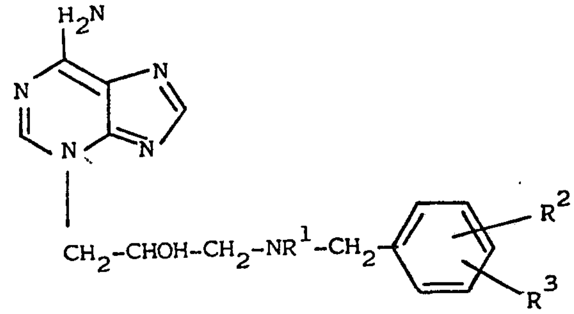

(II)

and salts thereof characterised in that $R^1$ is a hydrogen atom or a $C_{1-4}$ alkyl group; $R^2$ is a moiety selected from hydrogen, fluorine, chlorine and bromine atoms and $C_{1-4}$ alkyl, $C_{1-4}$ alkoxyl, $C_{1-4}$ alkanoyloxy, $C_{2-4}$ alkoxycarbonyl and trifluoromethyl groups; and $R^3$ is a moiety selected from hydrogen, fluorine and chlorine atoms and $C_{1-4}$ alkyl and $C_{1-4}$ alkoxyl groups.

2. A compound according to claim 1, characterised in that $R^1$ is a hydrogen atom or a methyl group.

3. A compound according to claim 1 or 2, characterised in that $R^2$ is a hydrogen atom and $R^3$ is a hydrogen or chlorine atom.

4. A compound according to claim 1 of the formula (I), or a salt thereof.

5. A compound according to claim 4, characterised in that $R^1$ is a hydrogen atom.

6. A compound according to claim 4 or 5, characterised in that $R^2$ is a hydrogen atom and $R^3$ is a hydrogen or chlorine atom.

7. 6-amino-9-[2-hydroxy-3-(N-benzyl-N-isopropylamino)propyl]purine or 6-amino-9-[2-hydroxy-3-(N-2′-chlorobenzylamino)propyl]purine or a salt thereof.

8. A pharmaceutical composition characterised by comprising a compound according to claim 1 of the formula (I) or (II) or a pharmaceutically acceptable salt thereof together with a pharmaceutically acceptable carrier.

9. A pharmaceutical composition characterised by comprising a compound according to claim 1 of the formula (I) or a pharmaceutically acceptable salt thereof together with a pharmaceutically acceptable carrier.

10. A process for the preparation of a compound according to claim 1, characterised by

a. the reaction of adenine or a metal salt thereof with a compound of the formula (III):

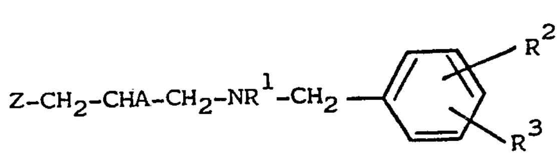

(III)

wherein:

$R^1$, $R^2$ and $R^3$ are as defined in claim 1 and

A is hydroxyl and Z is a group readily displaceable by a nucleophile; or

A and Z together form an oxide diradical;

b. the reaction of a compound of the formula (IV) or (V):

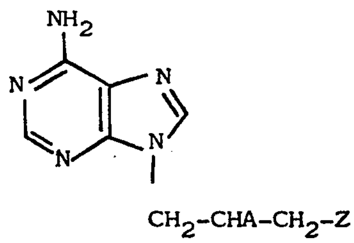

(IV)

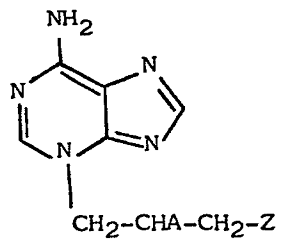

(V)

wherein:

A and Z are as hereinbefore defined with a compound of the formula (VI):

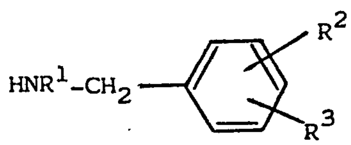

(VI)

wherein:

$R^1$, $R^2$ and $R^3$ are as defined in claim 1;

c. the reaction of a compound of the formula (VII) or (VIII):

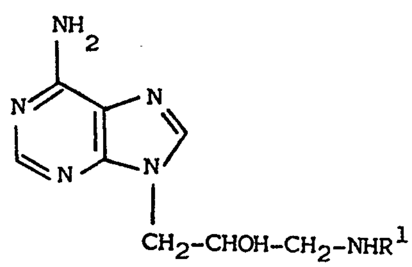

(VII)

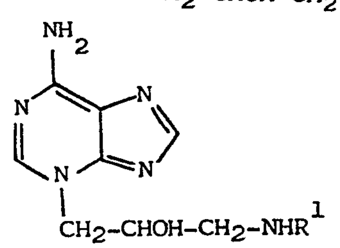

(VIII)

wherein $R^1$ is as hereinbefore defined, with a compound of the formula (IX):

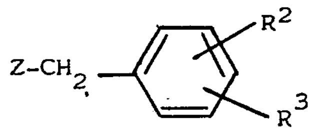

(IX)

wherein Z, $R^2$ and $R^3$ are as defined in formula (III);

d. the reaction of a compound of the formula (X) or (XI):

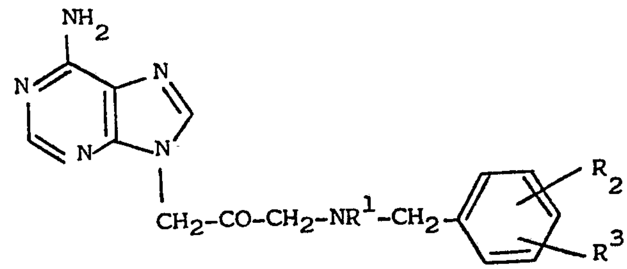

(X)

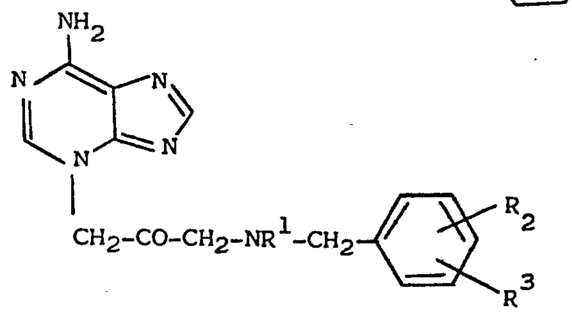

(XI)

wherein $R^1$, $R^2$ and $R^3{}_2$ are as defined in claim 1.

e. for a compound of the formula (I), the reaction of formamide with a compound of the formula (XII):

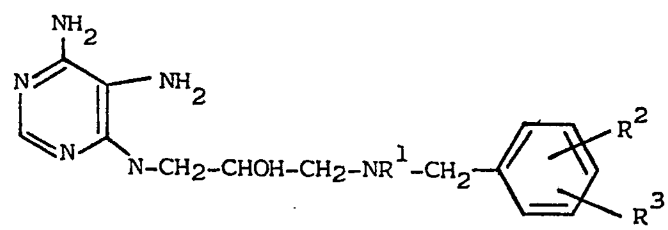

(XII)

wherein $R^1$, $R^2$ and $R^3$ are as defined in claim 1 and optionally thereafter salifying the compound of the formula (I) or (II) so produced; or converting $R^1$ when hydrogen to a $C_{1-4}$ alkyl group.

**Patentansprüche**

1. Eine Verbindung der Formel (I) oder (II):

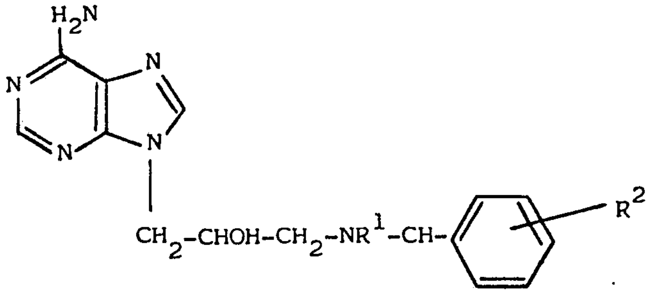

(I)

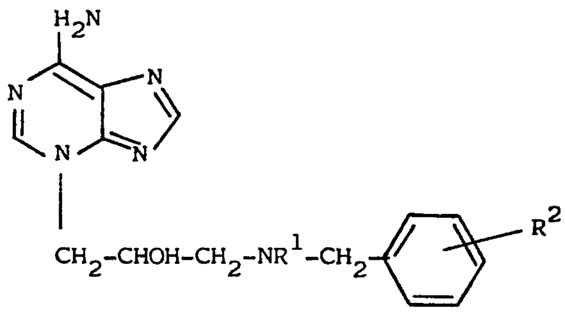

(II)

Und Salze derselben, dadurch gekennzeichnet, dass $R^1$ ein Wasserstoffatom oder eine $C_{1-4}$-alkyl-. gruppe ist, einen Molekuelteil bedeutet, ausgewaehlt aus Wasserstoff-, fluor-, chlor und Bromatomen und aus $C_{1-4}$-alkyl-, $C_{1-4}$-alkoxyl- und Trifluormethylgruppen., und $R^3$ ein Molekuelteil ist, ausgewaehlt aus Wasserstoff-, fluor- und Bromatomen und aus $C_{1-4}$-alkyl und $C_{1-4}$-alkoxylgruppen.

2. Eine Verbindung gemaess Anspruch 1, dadurch gekennzeichnet, dass $R^1$ ein Wasserstoffatom oder eine Methylgruppe ist.

3. Eine Verbindung gemaess Anspruch 1 oder 2, dadurch gekennzeichnet, dass $R^2$ ein Wasserstoffatom und $R^3$ ein Wasserstoff-oder Chloratom ist.

4. Eine Verbindung gemaess Anspruch 1 oder Formel (I) oder ein Salz derselben.

5. Eine Verbindung gemaess Anspruch 4, dadurch gekennzeichnet, dass $R^1$ ein Wasserstoffatom ist.

6. Eine Verbindung gemaess Anspruch 4 oder 5, dadurch gekennzeichnet, dass $R^2$ ein Wasserstoffatom und $R^3$ ein Wasserstoff-oder Chloratom ist.

7. 6-Amino-9-[2-hydroxy-3-(N-benzyl-N-isopropylamino)propyl]-purin oder 6-amino-9-[2-hydroxy-3-(N-2'-chlorbenzylamino)-propyl]Purin oder ein Salz dieser verbindungen.

8. Eine pharmazeutische Zubereitung, dadurch gekennzeichnet, dass sie eine Verbindung gemaess Anspruch 1 der Formel (I) oder (II) oder ein pharmakologisch vertraegliches Salz derselben zusammen mit einem pharmakologisch vertraeglichen Traeger enthaelt.

9. Eine pharmazeutisch Zubereitung, dadurch gekennzeichnet, dass sie eine Verbindung gemaess Anspruch 1 der Formel oder ein pharmakologisch vertraegliches Salz derselben zusammen mit einem pharmakologisch vertraeglichen Traeger enthaelt.

10. Ein Verfahren zur Herstellung einer Verbindung gemaess Anspruch 1 gekennzeichnet durch.

a) die Umsetzung von Adenin oder einem Metallsalz dieser Verbindung mit einer Verbindung der Formel (III):

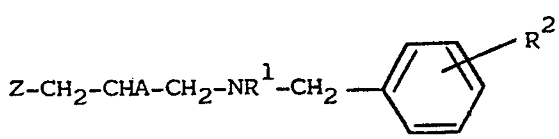

(III)

in welcher:

$R^1$, $R^2$ und $R^3$ wie in Anspruch 1 definiert sind und ein hydroxyl ist und Z eine leicht durch eine nukleophile Verbindung ersetzbare gruppe bedeutet, oder

A und Z zusammen ein oxid-diradikal bilden.

b) die Umsetzung einer Verbindung der Formel (IV) oder (V):

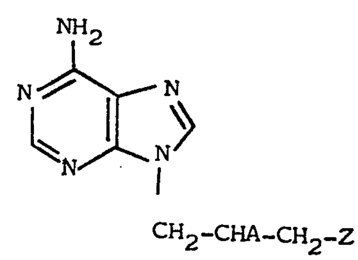

(IV)

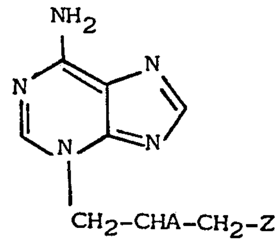

(V)

in der:
A und Z wie vorstehend sind, mit einer Verbindung der Formel (VI):

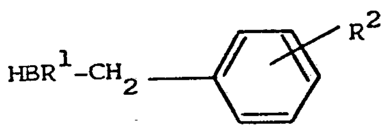

(VI)

in der:
$R^1$, $R^2$ und $R^3$ wie in Anspruch 1 definiert sind.

c) die Umsetzung einer Verbindung der Formel (VII) oder (VIII):

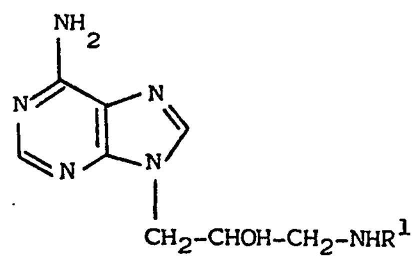

(VII)

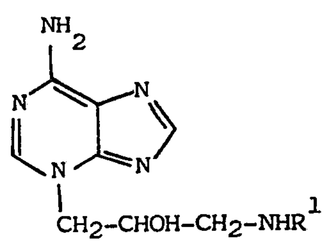

(VIII)

in welcher $R^1$ wie vorstehend definiert ist, mit einer Verbindung der Formel (IX):

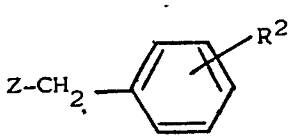

(IX)

in der, $R^2$ und $R^3$ wie in Formel (III) definiert sind.

d) die Umsetzung einer Verbindung der Formel (X) oder (XI):

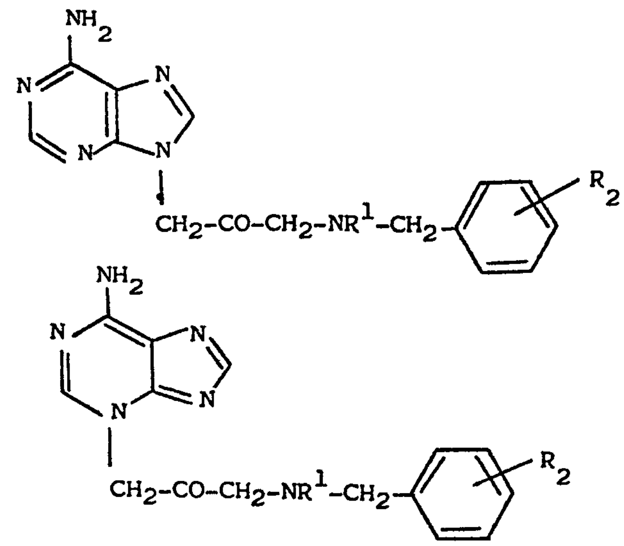

(X)

(XI)

in der $R^1$, $R^2$ und $R^3$ wie in Anspruch 1 definiert sind.

e) fuer eine Verbindung der Formel (I) durch die Umsetzung von Formamid mit einer Verbindung der Formel (XII)

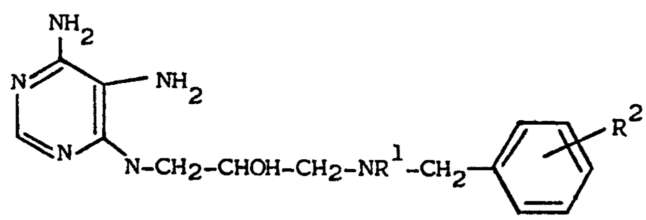

(XII)

in der $R^1$, $R^2$ und $R^3$ wie in Anspruch 1 definiert sind und gegebenenfalls anschliessende Bildung eines Salzes der so hergestellten Verbindung der Formel (I) oder (II) oder, falls $R^1$ ein Wasserstoffatom bedeutet, umwandlung in $C_{1-4}$-alkylgruppe.

**Revendications**

1. Composé de formule (I) ou (II):

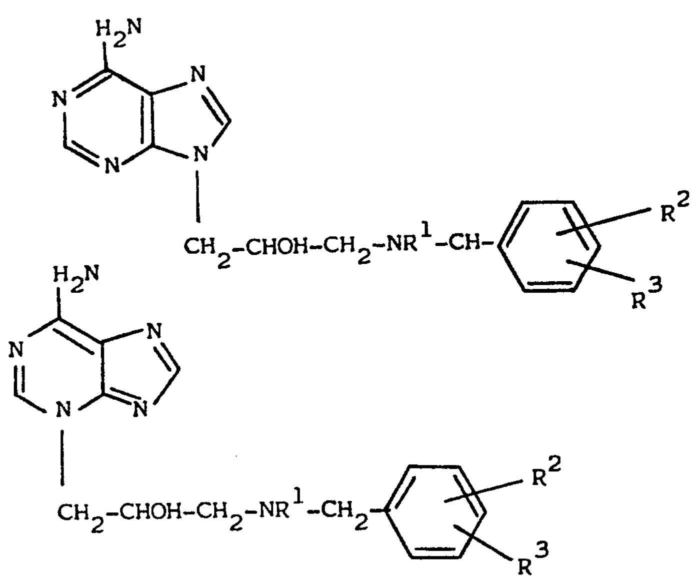

(I)

(II)

et ses sels, caractérisé en ce que $R^1$ est un atome d'hydrogène ou un groupe alcoyle en $C_{1-4}$; $R^2$ est une fraction molaire choisie parmi les atomes d'hydrogène, de fluor, de chlore et de brome et les groupes alcoyle en $C_{1-4}$, alcanoyloxy en $C_{1-4}$, alcoxycarbonyle en $C_{2-4}$ et trifluorométhyle; et $R^3$ est une fraction molaire choisie parmi les atomes d'hydrogène, de fluor et de chlore et les groupes alcoyle en $C_{1-4}$ et alcoxy en $C_{1-4}$.

2. Composé suivant la revendication 1, caractérisé en ce que $R^1$ est un atome d'hydrogène ou un groupe méthyle.

3. Composé suivant la revendication 1 ou 2, caractérisé en ce que $R^2$ est un atome d'hydrogène et $R^3$ est un atome d'hydrogène ou de chlore.

4. Composé suivant la revendication 1 de formule (I) ou sel de ce composé.

5. Composé suivant la revendication 4, caractérisé en ce que $R^1$ est un atome d'hydrogène.

6. Composé suivant la revendication 4 ou 5, caractérisé en ce que $R^2$ est un atome d'hydrogène et $R^3$ est un atome d'hydrogène ou de chlore.

7. 6-Amino-9-[2-hydroxy-3-(N-benzyl-N-isopropylamino)propyl]-purine ou 6-amino-9-[2-hydroxy-3-(N-2'-chlorobenzylamino)-propyl]purine ou sel de ceux-ci.

8. Composition pharmaceutique, caractérisée en ce qu'elle renferme un composé suivant la revendication 1 de formule (I) ou (II) ou un sel pharmaceutiquement acceptable de ce composé et un véhicule ou excipient pharmaceutiquement acceptable.

9. Composition pharmaceutique, caractérisée en ce qu'elle renferme un composé suivant la revendication 1 de formule (I) ou un sel pharmaceutiquement acceptable de ce composé, conjointement à un véhicule ou excipient pharmaceutiquement acceptable.

10. Procédé pour la préparation d'un composé suivant la revendication 1, caractérisé par

a. la réaction d'adénine ou d'un sel de métal de celle-ci avec un composé de formule (III):

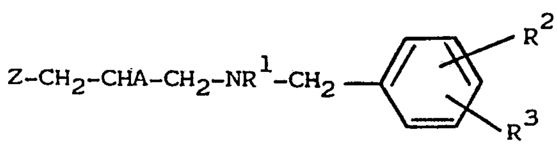

(III)

dans laquelle $R^1$, $R^2$ et $R^3$ ont la même signification que dans la revendication 1 et
A est un hydroxy et Z est un groupe aisément déplaçable par un nucléophile; ou
A et Z forment ensemble un diradical oxyde;

b. la réaction d'un composé de formule (IV) ou (V):

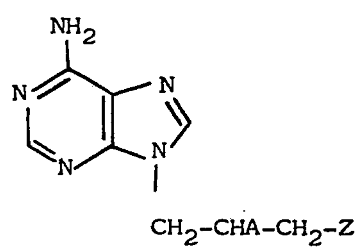

(IV)

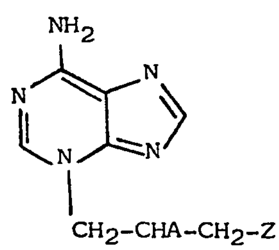

(V)

dans lesquelles
A et Z sont tels que définis ci-avant avec un composé de formule (VI):

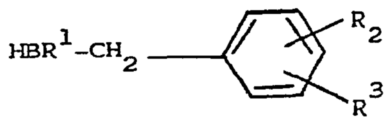

(VI)

dans laquelle $R^1$, $R^2$ et $R^3$ sont tels que définis dans la revendication 1;

c. la réaction d'un composé de formule (VII) ou (VIII):

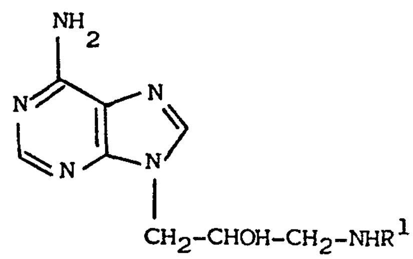

(VII)

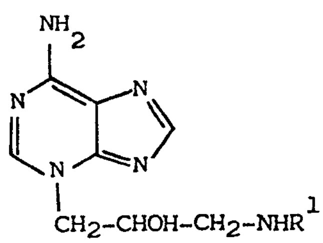

(VIII)

dans lesquelles $R^1$ est tel que défini ci-avant avec un composé de formule (IX):

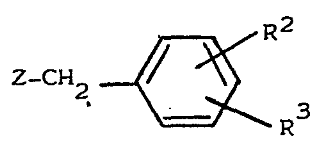

(IX)

dans laquelle Z, $R^2$ et $R^3$ sont tels que définis dans la formule (III):

d. La réduction d'un composé de formule (X) ou (XI):

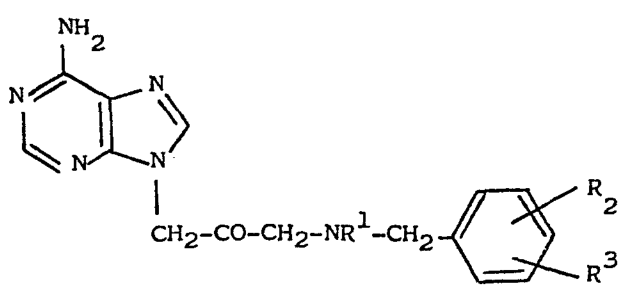

(X)

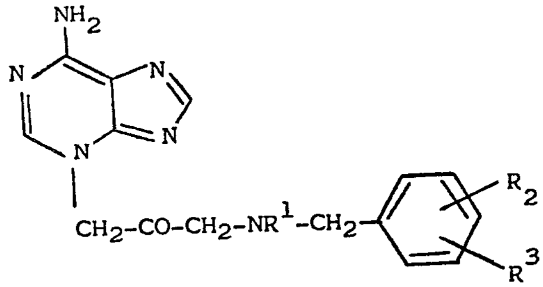

(XI)

dans lesquelles $R^1$, $R^2$ et $R^3$ sont tels que définis dans la revendication 1;

e. pour un composé de formule (I), la réaction de formamide avec un composé de formule (XII):

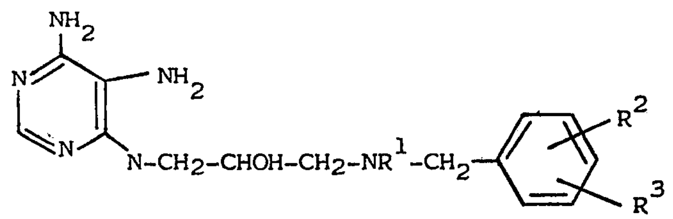

(XII)

dans laquelle $R^1$, $R^2$ et $R^3$ sont tels que définis dans la revendication 1, et éventuellement ensuite à salifier le composé de formule (I) ou (II) ainsi produit; ou à convertir $R^1$ quand il s'agit d'hydrogène en un groupe alcoyle en $C_{1-4}$.